# EUROPÄISCHE PATENTANMELDUNG

(11) **EP 2 769 748 A1**
(43) Veröffentlichungstag der Anmeldung: **27.08.2014**
(21) Anmeldenummer: 14000639.6
(22) Anmeldetag: 24.02.2014
(51) Int. Cl.: A61M 25/02, A44C 15/00

(54) **Fixiervorrichtung und deren Verwendung**

(30) Priorität: 25.02.2013 CH 505132013
(71) Anmelder: àWengen, Daniel F., 4103 Bottmingen (CH)
(72) Erfinder: àWengen, Daniel F., 4103 Bottmingen (CH)
(74) Vertreter: Rüedi, Regula Béatrice

(57) **Zusammenfassung**

Die vorliegende Anmeldung betrifft eine Vorrichtung (2) zum Anbringen an menschlichem oder tierischem Körpergewebe (1). Die Vorrichtung (2) weist hierbei einen ersten Teil und einen zweiten Teil auf, welche gegeneinander um eine Schwenkachse (3) schwenkbar sind. Der zweite Teil ist im Wesentlichen als eine um die Schwenkachse (3) gebogene Bogennadel (5) ausgebildet, welche zur Befestigung der Vorrichtung (2) am Körpergewebe (1) mittels einer Schwenkbewegung (S1) um die Schwenkachse (3) in das Körpergewebe (1) einstechbar ist. Ferner wird beispielhaft eine Verwendung für eine solche Vorrichtung (2) sowie eine die Vorrichtung (2) umfassende Anordnung gezeigt.

## Beschreibung

### Gebiet der Erfindung

Die Erfindung betrifft eine Vorrichtung zum Anbringen an menschlichem oder tierischem Körpergewebe. Ferner betrifft die Erfindung eine Verwendung der Vorrichtung sowie eine die Vorrichtung umfassende Anordnung.

### Hintergrund

Ein gängiges Problem ist das Anbringen und Befestigen an menschlichem oder tierischem Körpergewebe, beispielsweise infolge der bekannten, flexiblen und miteinander verbundenen Zell- bzw. Gewebeschicht- und Hauteigenschaften, der Verletzlichkeit und Empfindlichkeit des Körpergewebes bzw. der Gewebeschichten sowie der Haut oder der Blutbahnen.

Besonders umständlich und zeitaufwändig ist es auch, Objekte wie z.B. Röhrchen, Schläuche etc. am Körpergewebe anzubringen. In der Regel wird hierzu, z.B. unter Zuhilfenahme einer Bogennadel, ein an einem Ende der Bogennadel befestigter Faden durch das Körpergewebe gestossen. Dieser tritt üblicherweise an einer von einer Eintrittsstelle beabstandeten Stelle wieder aus dem Körpergewebe aus. Die Bogennadel mit daran befestigtem Faden sollte dabei nicht zu tief in das Körpergewebe hindurch gestossen werden, um unnötige Verletzungen am Körpergewebe zu vermeiden. Beide vom Körpergewebe wegragende Fadenenden müssen sodann so gegeneinander verschoben werden, dass die Enden eine genügende Minimallänge zum späteren, in der Regel mehrmaligen, Verknoten aufweisen.

Sodann wird der Faden ein erstes Mal gegen Verrutschen gesichert, üblicherweise durch Verknoten der beiden Fadenenden mittels wenigstens einem Sicherungsknoten. Dieser Sicherungsknoten kommt vorzugsweise ein wenig beabstandet vom Körpergewebe zu liegen und wird nicht zu stark angezogen. Beides wiederum zur Vermeidung von Verletzungen am Körpergewebe und auch zur Vermeidung eines unerwünschten Fadenrisses, wodurch die Prozedur wiederholt werden müsste.

Schliesslich wird ein gewünschtes Objekt, z.B. ein Röhrchen oder ein Schlauch, zwecks Befestigung am Körpergewebe zwischen die beiden Fadenenden und auf der vom Körpergewebe beabstandeten Seite des/der Sicherungsknoten eingelegt und mit den beiden freien Fadenenden einfach oder mehrfach verknotet. Das Objekt, ggf. auch mehrere Objekte, werden nach einiger Zeit, z.B. nach einigen Stunden oder Tagen, wieder entfernt. Der Faden kann hierbei ein sich mit der Zeit im Körpergewebe auflösender Faden sein und darf deshalb im Körpergewebe verbleiben oder er wird aus dem Körpergewebe entfernt.

### Darstellung der Erfindung

Der Erfindung liegt die Aufgabe zu Grunde, eine Vorrichtung zu schaffen, welche einfach zu handhaben ist, auf sichere und rasche Weise an menschlichem oder tierischem Körpergewebe anbringbar und entfernbar ist und gleichzeitig Verletzungen am Körpergewebe infolge Anbringen und/oder Entfernen auf ein Minimum reduziert.

Diese Aufgabe wird in Bezug auf die Vorrichtung mit den Merkmalen des Anspruchs 1 gelöst. Hiernach weist die Vorrichtung einen ersten Teil und einen zweiten Teil auf, welche gegeneinander um eine Schwenkachse schwenkbar sind. Dabei ist der zweite Teil im Wesentlichen als eine um die Schwenkachse gebogene Bogennadel ausgebildet. Die Bogennadel ist dabei zur Befestigung der Vorrichtung am Körpergewebe mittels einer Schwenkbewegung um die Schwenkachse in das Körpergewebe einstechbar.

Das Körpergewebe kann hierbei menschlich oder tierisch sein und z.B. lebend, betäubt oder abgestorben. Die aufgrund der geometrischen Verhältnisse und der Grössenverhältnisse der Vorrichtung mittels definierter Schwenkbewegung um die Schwenkachse und gleichzeitig mit definierter Eindringtiefe und mit definiert voneinander beabstandeten Eintritts- und Austrittsstellen in das Körpergewebe eingebrachte bzw. eingestochene und von der Rückseite des Körpergewebes wieder austretende Bogennadel sichert nun die Vorrichtung im Körpergewebe. Selbst vom Körpergewebe in einem beliebigen Winkel weggerichtete Zugkräfte werden von der im Körpergewebe eingebrachten Bogennadel aufgenommen bzw. derart in das Körpergewebe geleitet, dass die Bogennadel bzw. die Vorrichtung nicht ausreisst, wodurch Verletzungen am Körpergewebe vermieden werden.

Die geometrischen Verhältnisse und die Grössenverhältnisse der Vorrichtung geben wie erwähnt den Schwenkradius und damit die Eindringtiefe sowie die Abstände zwischen Eintritts- und Austrittsstelle vor. Mit anderen Worten ist das Anbringen bzw. Einstechen und spätere Entfernen der Vorrichtung bzw. der Bogennadel vom/aus dem Körpergewebe ein durch deren zweiteilige, bogenförmige Konstruktion ermöglichter, um die Schwenkachse geführter und daher reproduzierbarer Vorgang. Dieser ist sehr einfach zu handhaben. Ausserdem erlaubt die erfindungsgemässe Vorrichtung ein rasches Anbringen und Entfernen vom Körpergewebe, d.h. innert wenigen Sekunden oder Sekundenbruchteilen. Dennoch lassen sich Verletzungen am Hautgewebe bzw. in Gewebeschichten weitgehend vermeiden bzw. auf ein Minimum an den Einstich- (bzw. Eintritts-) und Austrittsstellen der Bogennadel reduzieren. Bei sachgemässem Anbringen oder Entfernen der Vorrichtung bzw. der Bogennadel ist das Risiko von Verletzungen am oder im Körpergewebe daher sehr klein.

In einer anderen vorteilhaften Variante ist die Vorrichtung als ein um die Schwenkachse gebogenes Führungsrohr ausgebildet, welches mindestens einen Teil der Bogennadel in einer ersten Schwenkposition umschliesst.

In einer weiteren, vorteilhaften Variante erstreckt sich die Bogennadel über einen Winkelbereich von mindestens 180° um die Schwenkachse. Hierdurch ist es ermöglicht, dass beide Enden der in das Körpergewebe eingebrachten Bogennadel in ihrer Endposition oder in einer Zwischenposition an den Einstich- und Austrittsstellen gleichzeitig aus dem Körpergewebe hervortreten bei gleichzeitig minimaler Einstichtiefe durch die Bogennadel. Die Bogennadel und/oder das Führungsrohr ist/sind hierbei je nach Ausführung entlang der Schwenkachse und/oder in einem Rohrquerschnitt z.B. rund, abgerundet oder oval ausgebildet. Ein Schwenkradius ist in der gezeigten Ausführungsform konstant ausgebildet, kann aber abhängig vom Einsatzzweck in anderen Ausführungsformen auch nicht konstant sein.

In einer nochmals anderen, besonders vorteilhaften Ausführung sind zudem am ersten Teil der Vorrichtung Mittel zum Befestigen von Objekten ausgebildet. Hierbei sind die Mittel vorzugsweise als wenigstens je eine Öse, eine Bride, ein Faden, ein federnder Greifarm, ein Klettband und/oder als eine Klammer ausgeformt. Die Mittel sind vorteilhaft zur Befestigung von Schläuchen oder Röhrchen ausgestaltet.

Diese Mittel ermöglichen somit das einfache, sichere und rasche Befestigen der Objekte an der Vorrichtung und damit am Körpergewebe. Möglicherweise entstehende, vom Körpergewebe weggerichtete und an die Vorrichtung angreifende Zugkräfte werden durch die Bogennadel wie erwähnt derart in das Körpergewebe geleitet, dass bei fachgerechter Handhabung der Vorrichtung ein Ausreissen der Bogennadel unterbleibt und daraus resultierende Verletzungen am Körpergewebe vermieden werden.

In einer weiteren vorteilhaften Ausführungsform ist an der Bogennadel ein Griff angeordnet, der sich durch einen Schlitz im ersten Teil erstreckt und mit welchem die Bogennadel verschwenkbar ist. Der Griff ragt z.B. teilweise oder ganz durch den Schlitz hindurch und ist von aussen bedienbar. In einer anderen Ausführungsvariante ist der Griff als Schieber ausgeformt, z.B. mit einer vorteilhaft rauen oder geriffelten und der/den Fingerkuppe/n angepassten Berührungsfläche bzw. Schiebefläche. Der Schieber ist je nach Variante im Schlitz ganz oder teilweise versenkt und durch diesen hindurch oder von aussen bedienbar. Die raue oder geriffelte Berührungsfläche hilft, ein Abrutschen der/des Finger/s beim Vor- oder Rückwärtsschwenken der Bogennadel zu vermeiden. Der Schlitz wiederum erstreckt sich über nahezu die gesamte Länge am bogenäusseren Umfang bzw. entlang einer Längsmittelachse des ersten Teils der Vorrichtung, welche wie erwähnt als Führungsrohr ausgebildet sein kann.

In einer weiteren Variante weist die Bogennadel ein erstes, zugespitztes Ende und ein dem ersten Ende gegenüberliegendes zweites Ende auf, wobei der Griff am zweiten Ende angeordnet ist. Der Griff kann hierbei wie erwähnt auch als Schieber ausgeführt sein und das zugespitzte Ende ist derart als Bogennadelspitze ausgeformt, dass sie sich eignet, um einen Widerstand des Körpergewebes bzw. der Bogennadelspitze entgegen wirkenden Hautspannung zu überwinden und in das Körpergewebe einzudringen.

In einer nochmals anderen Variante ist die Bogennadel mit der Schwenkbewegung von einer Anfangsposition in eine Endposition überführbar, wobei die Bogennadel in der Anfangsposition und/oder in der Endposition gegen ein Verschwenken verriegel- oder sicherbar ist. Zudem steht in einer weiteren Variante die Bogennadel (5) in der Anfangs- und Endposition (P2, P3) über den ersten Teil vor und insbesondere weist die Bogennadel (5) weiter eine Sicherungsposition (P1, P2, P3) auf, in welcher sie sicherbar ist, und wobei die Spitze (11) der Bogennadel (5) in der Sicherungsposition (P1) nicht über den ersten Teil vorsteht.

Die Verriegelung oder Sicherung in einer der Positionen ist in einer vorteilhaften Variante mechanisch ausgeformt, z.B. derart, dass ein Haken am zweiten Teil in eine Öse am ersten Teil (oder umgekehrt) oder ein Haken am zweiten Teil an eine vorstehende Nase am ersten Teil (oder umgekehrt) einhängbar ist. Alternativ oder ergänzend ist auch eine Verriegelung bzw. Sicherung mittels Sicherungsstift durch eine Öffnung, welche eine Bohrung sein kann, durch beide Teile hindurch ausgebildet. Auch ein stufenloses Sichern/Verriegeln der Bogennadel z.B. mittels einer von Hand anzieh- und lösbaren Schraube oder mittels einem verriegelbaren Griff bzw. Schieber ist in weiteren Varianten ausgebildet.

Erfolgt die Verriegelung in einer ersten Anfangsposition, ist die Bogennadel bzw. Bogennadelspitze wie erwähnt nicht aus dem ersten Teil der Vorrichtung hervorstehend, welche ebenfalls wie erwähnt vorteilhaft als Führungsrohr ausgebildet ist. Verletzungen an der (nicht vorstehenden) Bogennadelspitze sind somit in dieser ersten, verriegelten bzw. gesicherten Anfangsposition ausgeschlossen.

Erfolgt die Verriegelung in einer zweiten Anfangsposition, so ragt die Bogennadelspitze genügend weit aus dem ersten Teil der Vorrichtung heraus, so dass die Bogennadelspitze im Moment des Einstechens sicher, gezielt und genügend tief in die Oberfläche des Körpergewebes einstechbar ist, ohne dass die Bogennadel durch die entgegengesetzt wirkenden Kräfte in die erste Anfangsposition zurückgleitet. Einer der Vorteile liegt somit darin, dass der grösste Widerstand, welcher im Moment des Einstechens auftritt, beim Anbringen der Vorrichtung zielgenau, rasch, sicher und mit wenig Kraftaufwand überwindbar ist. Auch in der Endposition oder allen zwischen zweiter Anfangs- und Endposition liegenden Positionen ragt die Bogennadelspitze aus dem Führungsrohr heraus.

In einer weiteren Ausführungsform weist der erste Teil der Vorrichtung wenigstens einen Anschlag für die Bogennadel auf, vorzugsweise in der Anfangsposition und/oder Endposition. Mit der Anfangsposition ist in einer bevorzugten Variante die vorgängig erwähnte erste Anfangs- bzw. die Ausgangsposition der Bogennadel beschrieben; und mit Endposition eine letzte Position der Bogennadel, wobei sich die Bogennadelspitze nun von einer Gewebeinnenseite her durch das Körpergewebe hindurch gestochen an der Austrittsstelle des Körpergewebes befindet.

In einer weiteren Variante erstreckt sich die Bogennadel auf einem Kreissegment um die Schwenkachse, wodurch eine geführte Schwenkbewegung nach dem Einstechen der Bogennadel von der zweiten Anfangsposition bis zur Endposition ermöglicht ist. Die Schwenkbewegung kann dabei wie erwähnt kreis- oder bogenförmig sein.

Die Vorrichtung oder einzelne Teile der Vorrichtung sind in vorteilhaften Ausführungsformen aus Kunststoff, rostfreiem Stahl und/oder Edelmetall gefertigt. Das Material ist je nach Ausführungsform und Verwendungszweck der Vorrichtung als ein Wegwerfartikel konzipiert oder z.B. auch als ein Material, dass sich zur Sterilisation bzw. Entkeimung eignet. Die Materialwahl erfolgt in wieder anderen Varianten oder Verwendungszwecken vornehmlich aus Gründen der Ästhetik und des Designs und damit letztlich aus Gründen der Formschönheit.

In Bezug auf die Verwendung der Vorrichtung wird die Aufgabe dadurch gelöst, dass die Vorrichtung als ein Piercing oder zur Befestigung eines Objekts an menschlichem oder tierischem Körpergewebe verwendbar ist. Die Vorrichtung eignet sich z.B. zum Piercing an Zunge, Lippe, Augenbraue oder Bauchnabel und ist vorteilhaft als Schmuckstück ausgeformt.

In einer anderen Ausführungsform sind wie erwähnt am ersten Teil der Vorrichtung auch Mittel zum Befestigen von Objekten ausgebildet, z.B. je eine Öse, eine Bride, ein Faden und/oder ein federnder Greifarm. Die Vorrichtung eignet sich in einer solchen Ausführungsform also auch bestens zur Verwendung als Befestigungsmittel für Objekte wie z.B. Schläuche oder Röhrchen an menschlichem oder tierischem Körpergewebe. Dies insbesondere, weil wie erwähnt die in einem beliebigen Winkel vom Körpergewebe weggerichteten Zugkräfte durch die darin eingebrachte Bogennadel derart aufnimmt bzw. in das Körpergewebe leitet, dass die Bogennadel bzw. die Vorrichtung nicht ausreisst.

In Bezug auf die die Vorrichtung umfassende Anordnung wird die Aufgabe dadurch gelöst, dass die Anordnung eine Vorrichtung sowie mindestens einen an dieser mit Befestigungsmitteln befestigten Schlauch und/oder mindestens ein befestigtes Röhrchen zum Ableiten oder Zuleiten eines Fluids aus einem/in einen Körper aufweist, wobei der Schlauch oder das Röhrchen vorzugsweise als ein Blutungsdrain, ein Bülaudrain, eine PEG-Sonde, eine suprapubische Ableitung oder eine Wunddrainage nach einem chirurgischen Eingriff, oder als eine andere Drainage ausgebildet ist. Der erwähnte Blutungsdrain, Bülaudrain (Drain z. B für eine Lunge) bzw. die erwähnte PEG-Sonde (PEG = Perkutane endoskopische Gastrostomie, Sonde z.B. für einen Magen), die erwähnte suprapubische Ableitung (z.B. Ableitung für eine Urinblase), oder die erwähnte Wunddrainage oder andere Drainage ist/sind in vorteilhaften Ausführungsformen aus Silikon ausgebildet und dem Fachmann bekannt, weshalb sie keine weiteren Ausführungen bedürfen.

In einer bevorzugten Anordnung ist der Schlauch und/oder das Röhrchen an dessen äusseren Oberfläche wenigstens in einem Befestigungsbereich aufgeraut und/oder mit Kerben, Vertiefungen oder Wölbungen ausgebildet, wobei der Schlauch oder das Röhrchen mit dem Befestigungsmittel am Befestigungsbereich gehalten ist. Ein unbeabsichtigtes Verschieben/Verrutschen des Schlauchs oder Röhrchens, z.B. infolge einer versehentlichen, unerwünschten und z.B. ruckartigen Bewegung eines Patienten und/oder eines Arztes/einer Pflegefachperson, und eine hierdurch möglicherweise auftretende Verletzung, wird durch eine solche Anordnung erheblich reduziert.

### Kurze Beschreibung der Zeichnungen

Weitere Ausgestaltungen, Vorteile und Anwendungen der Vorrichtung sowie eine bevorzugte Verwendung der Vorrichtung und der Anordnung ergeben sich aus den abhängigen Ansprüchen und aus der folgenden Beschreibung von bevorzugten Ausführungsbeispielen anhand der Figuren. Dabei zeigen:
Fig. 1 eine Vorrichtung mit einem um eine Schwenkachse gebogenen Führungsrohr und einer Bogennadel in einer zweiten Anfangsposition,
Fig. 2 eine Vorrichtung gemäss Fig. 1 in einer Endposition, an welcher zudem eine Bride zum Befestigen von Objekten ausgebildet ist mit einem daran befestigten Schlauch,
Fig. 3 eine Verwendung der Vorrichtung als ein Piercing an einer Augenbraue, und
Fig. 4 eine weitere Verwendung der Vorrichtung als Fixation in einem Körpergewebe für einen Schlauch, ein Röhrchen, eine Drainage oder ähnlich.

### Wege zur Ausführung der Erfindung

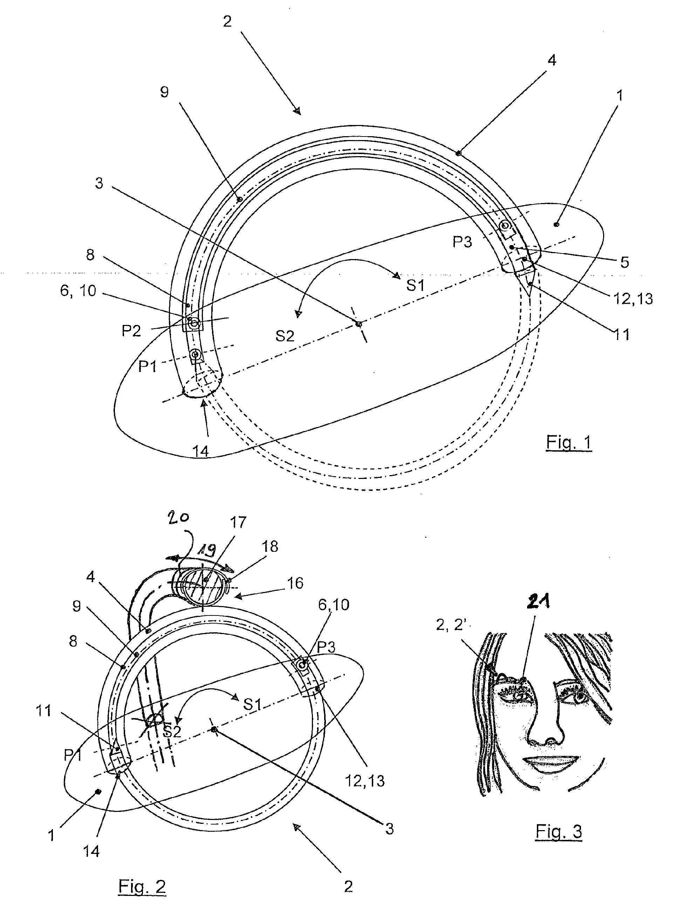

Fig. 1 zeigt eine an menschlichem oder tierischem Körpergewebe 1 angebrachte Vorrichtung 2 mit einem um eine Schwenkachse 3 gebogenen Führungsrohr 4 als erstem Teil und einer Bogennadel 5 als zweitem Teil. Die Bogennadel 5 ist hierbei durch einen als Sicherungsschraube ausgeformten Sicherungsgriff 6 stufenlos in einer zweiten Anfangs- bzw. Schwenkposition P2 gesichert. Ein nicht dargestellter Gewindebolzen am Griff 6 ragt hierbei durch einen Schlitz 8 im Führungsrohr 4 durch dieses hindurch und ist mittels ebenfalls nicht dargestelltem Gewinde in der Bogennadel 5 von Hand verschraubt. Der Schlitz 8 erstreckt sich über nahezu die gesamte Länge einer Längsmittelachse 9 des Führungsrohrs 4, wobei sich die Längsmittelachse 9 und die Schwenkachse 3 überdecken. Ein Kopf 10 am Griff 6 übernimmt dabei eine Klemmfunktion vergleichbar mit einem Schraubenkopf, wodurch Führungsrohr 4 und Bogennadel 5 relativ zueinander gegen ein bogenförmiges Verschwenken entlang der Schwenkachse 3 bzw. de Längsmittelachse 9 in beide Richtungen der Schwenkbewegung S1, S2 gesichert sind.

Gestrichelt angedeutet sind zudem eine erste Anfangs- bzw. Schwenkposition P1 und eine End- bzw. Schwenkposition P3 des Griffs 6 bzw. der Bogennadel 5, wobei die Bogennadel 5 ebenfalls in beide Richtungen gegen eine Schwenkbewegung S1, S2 gesichert ist. In der Anfangsposition P1 ist eine Bogennadelspitze 11 vollständig und verletzungssicher im Führungsrohr 4 eingeschoben. Dagegen ragt die Bogennadelspitze 11 in der gesicherten zweiten Anfangsposition P2 aus einer Querschnittsöffnung bzw. einer Austrittsöffnung 12 des Führungsrohrs 4 heraus und ist gleichzeitig an einer Eintrittstelle 13 am Körpergewebe 1 soweit in das Körpergewebe 1 eingestochen, dass das Führungsrohr 4 auf dem Körpergewebe 1 aufsitzt.

In der Endposition P3 schliesslich tritt die Bogennadel 11 nach einer bogenförmig geführten Schwenkbewegung S1 sodann in eine Querschnittsöffnung bzw. Eintrittsöffnung 14 des Führungsrohrs 4 rohrinnenseitig ein. Die Eintrittsöffnung 14 führt die eingeführte Bogennadelspitze 11 bzw. Bogennadel 5 und nimmt diese derart auf, dass die Bogennadel 5 und das Führungsrohr 4 einen geschlossenen und gesicherten Ring bilden mit entsprechend stabilen Eigenschaften. Die Bogennadelspitze 11 ist dabei vollständig und wiederum verletzungssicher durch die Öffnung 14 im Führungsrohr 4 eingeschoben. Die Bogennadel 5 leitet auf diese Weise wie erwähnt selbst vom Körpergewebe 1 in einem beliebigen Winkel weggerichtete Zugkräfte derart in das Körpergewebe 1 ein, dass die Bogennadel 5 bzw. die Vorrichtung 2 nicht ausreisst.

In der gezeigten Ausführungsvariante begrenzt ein nicht dargestellter Anschlag die Bogennadel 5 an der ersten Anfangsposition P1 und an der Endposition P3 am Anfang bzw. Ende der beiden Schwenkbewegungen S1, S2.

Die Bogennadelspitze 11 ist in einer weiteren, nicht dargestellten Ausführungsform austauschbar. Unterschiedliche Bogennadelspitzen 11 sind somit abgestimmt auf die jeweiligen Körpergewebeeigenschaften und -dicken, insbesondere bzgl. des Widerstands beim Einstechen der Bogennadel an der Eintrittstelle, mit einer einzelnen Vorrichtung 2 einsetzbar.

**Fig. 2** zeigt eine Vorrichtung 2 gemäss Fig. 1 in einer Endposition P3. An der Vorrichtung 2 ist als ein Befestigungsmittel insbesondere eine Bride 16 vorgesehen zum Befestigen von Objekten wie z.B. einem Röhrchen oder einem Schlauch 17, wobei in der gezeigten Variante ein Schlauch 17 an der Bride 16 befestigt ist. Der Schlauch 17 ist in der gezeigten Ausführungsvariante als ein aus einem Körper durch das Körpergewebe 1 herausführender Blutungsdrain aus Silikon ausgebildet und in einem Klemm- bzw. Befestigungsbereich 19 der besseren Übersichtlichkeit wegen geschnitten dargestellt. Die Bride 16 klemmt hierbei den Schlauch 17 mit einem Klemmelement fest, welches im gezeigten Beispiel als Gummiband 18 ausgebildet ist. Hierbei ist der Schlauch 17 im Befestigungsbereich 19 zudem mit einigen Kerben 20 ausgebildet, welche den Schlauch 17 beim Festklemmen zusätzlich gegen ein unbeabsichtigtes Verschieben/Verrutschen sichert, das wie erwähnt z.B. infolge einer versehentlichen, unerwünschten und z.B. ruckartigen Bewegung eines Patienten und/oder eines Arztes/einer Pflegefachperson hervorgerufen werden könnte. Durch die gezeigte Anordnung werden somit die mit diesem unerwünschten Verschieben/Verrutschen möglicherweise auftretenden Verletzungen z.B. im Körpergewebe ausgeschlossen. Des Weiteren sind die Kerben 20 derart ausgebildet, dass sie nicht aus dem Schlauch 17 bzw. dessen Oberfläche vorstehen und hierdurch z.B. das Einführen/ Ausführen der Blutungsdrains in/aus dem Körpergewebe 1 nicht erschweren. In anderen, nicht dargestellten Anordnungen werden auch Schläuche oder Röhrchen genutzt, die nicht nur im Klemmbereich, sondern auf der gesamten Oberfläche aufgeraut und/oder mit Kerben (20), Vertiefungen oder Wölbungen ausgebildet sind, welche symmetrisch oder asymmetrisch verteilt und vorteilhaft nicht vorstehend angeordnet sind.

In weiteren, nicht gezeigten Varianten sind auch mehrere Objekte gleichzeitig an der Vorrichtung 2 bzw. am Körpergewebe 1 anbringbar, entweder mit einem gemeinsamen Befestigungsmittel oder mehreren separaten Befestigungsmitteln.

**Fig. 3** zeigt eine beispielhafte Verwendung der Vorrichtung 2 (ohne Mittel zum Befestigen von Objekten) als ein Piercing 2' an einer Augenbraue 21. Die Vorrichtung 2 bzw. das Piercing 2' findet somit als ein Schmuckstück Verwendung und ist z.B. aus einem Edelmetall angefertigt, vorzugsweise Gold oder Silber, oder auch andere körperverträgliche Metalle wie z.B. Titan oder Edelstahl.

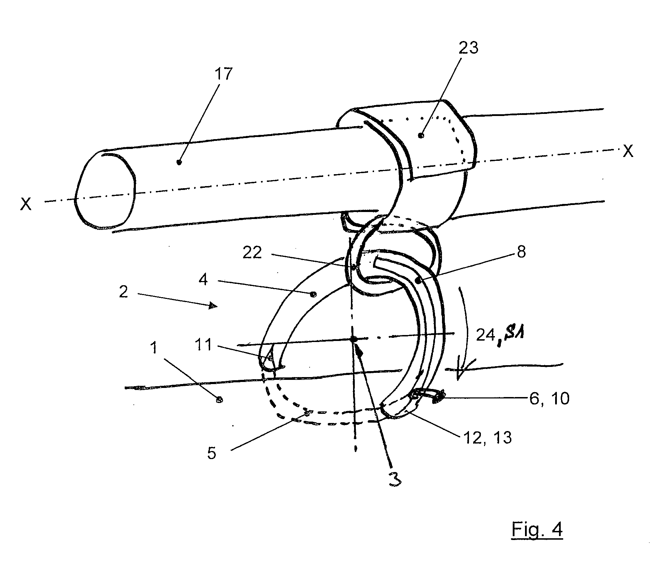

**Fig. 4** zeigt beispielhaft eine weitere Verwendung der Vorrichtung 2 als Fixation in einem Körpergewebe 1 für den Schlauch 17, ein Röhrchen, eine Drainage oder ähnlich. Die Vorrichtung 2 ist hierbei besonders vorteilhaft angeordnet mit einem ersten als Öse 22 und einem zweiten als Klettband 23 ausgebildeten Befestigungsmittel, mit welchen der Schlauch 17 oder das Röhrchen/die Drainage zum Ableiten oder Zuleiten eines Fluids aus einem/in einen Körper befestigt ist. Die Vorrichtung 2 ist hierbei im Körpergewebe 1 eingestochenen und in Schliessrichtung 24 geschlossen und verriegelt dargestellt. In anderen, nicht dargestellten Ausführungsvarianten sind auch zwei oder mehr Schläuche 17 und/oder Röhrchen/Drainagen mit Befestigungsmitteln an der Vorrichtung 2 befestigt. Der Schlauch 17 oder das Röhrchen sind hierbei wie erwähnt vorteilhaft und je nach Verwendungszweck als ein Blutungsdrain, ein Bülaudrain, eine PEG-Sonde, eine suprapubische Ableitung oder eine Wunddrainage nach einem chirurgischen Eingriff, oder als eine andere Drainage ausgebildet.

Die dargestellte Ausführung ermöglicht dank allgemein bekanntem Klettverschluss 23 ein einfaches und rasches Befestigen des Schlauchs 17 oder Röhrchens/der Drainage in der gewünschten Position entlang einer Schlauch- oder Rohrlängsmittelachse X-X. In einer weiteren, nicht dargestellten Ausführungsform ist wenigstens ein Schlauch 17 und/oder wenigstens ein Röhrchen/eine Drainage direkt, d.h. ohne eine dazwischen angeordnete Öse 22, mit wenigstens einem Klettband 23 an der Vorrichtung 2 befestigt.

## Patentansprüche

1. Vorrichtung (2) zum Anbringen an menschlichem oder tierischem Körpergewebe (1), **dadurch gekennzeichnet, dass** die Vorrichtung (2) einen ersten Teil und einen zweiten Teil aufweist, welche gegeneinander um eine Schwenkachse (3) schwenkbar sind, wobei der zweite Teil im Wesentlichen als eine um die Schwenkachse (3) gebogene Bogennadel (5) ausgebildet ist, welche Bogennadel (5) zur Befestigung der Vorrichtung (2) am Körpergewebe (1) mittels einer Schwenkbewegung (S1) um die Schwenkachse (3) in das Körpergewebe (1) einstechbar ist.

2. Vorrichtung (2) nach Anspruch 1, wobei der erste Teil der Vorrichtung (2) als ein um die Schwenkachse (3) gebogenes Führungsrohr (4) ausgebildet ist, welches mindestens einen Teil der Bogennadel (5) in einer ersten Schwenkposition (P1, P2) umschliesst.

3. Vorrichtung (2) nach Anspruch 1 oder 2, wobei sich die Bogennadel (5) über einen Winkelbereich von mindestens 180° um die Schwenkachse (3) erstreckt.

4. Vorrichtung (2) nach einem der vorangegangenen Ansprüche, wobei am ersten Teil Mittel zum Befestigen von Objekten ausgebildet sind, wobei die Mittel vorzugsweise als wenigstens je eine Öse (22), eine Bride (16), ein Faden, ein federnder Greifarm, ein Klettband (23) und/oder als eine Klammer ausgeformt sind.

5. Vorrichtung (2) nach Anspruch 4, wobei die Mittel zum Befestigen ausgestaltet sind zur Befestigung von Schläuchen (17) oder Röhrchen.

6. Vorrichtung (2) nach einem der vorangegangenen Ansprüche, wobei an der Bogennadel (5) ein Griff (6) angeordnet ist, der sich durch einen Schlitz (8) im ersten Teil erstreckt und mit welchem die Bogennadel (5) verschwenkbar ist.

7. Vorrichtung (2) nach Anspruch 6, wobei die Bogennadel (5) ein erstes, zugespitztes Ende (11) und ein dem ersten Ende gegenüberliegendes zweites Ende aufweist, wobei der Griff (6) am zweiten Ende angeordnet ist.

8. Vorrichtung (2) nach einem der vorangegangenen Ansprüche, wobei die Bogennadel (5) mit der Schwenkbewegung (S1) von einer Anfangsposition (P1, P2) in eine Endposition (P3) überführbar ist, und wobei die Bogennadel (5) in der Anfangsposition (P1, P2) und/oder in der Endposition (P3) gegen ein Verschwenken verriegel- oder sicherbar ist.

9. Vorrichtung (2) nach Anspruch 8, wobei die Bogennadel (5) in der Anfangs- und Endposition (P2, P3) über den ersten Teil vorsteht und insbesondere, wobei die Bogennadel (5) weiter eine Sicherungsposition (P1, P2, P3) aufweist, in welcher sie sicherbar ist, und wobei die Spitze (11) der Bogennadel (5) in der Sicherungsposition (P1) nicht über den ersten Teil vorsteht.

10. Vorrichtung (2) nach einem der vorangegangenen Ansprüche, wobei der erste Teil der Vorrichtung (2) wenigstens einen Anschlag für die Bogennadel (5) aufweist, vorzugsweise in der Anfangs- (P1) und/oder Endposition (P3).

11. Vorrichtung (2) nach einem der vorangehenden Ansprüche, wobei sich die Bogennadel (5) auf einem Kreissegment um die Schwenkachse (3) erstreckt.

12. Verwendung der Vorrichtung (2) nach einem der Ansprüche 1 bis 11 als ein Piercing (2') oder zur Befestigung eines Objekts an menschlichem oder tierischem Körpergewebe (1).

13. Anordnung umfassend eine Vorrichtung (2) nach einem der Ansprüche 1 bis 11 sowie mindestens einen an dieser mit Befestigungsmitteln befestigten Schlauch (17) und/oder mindestens ein befestigtes Röhrchen zum Ableiten oder Zuleiten eines Fluids aus einem/in einen Körper, wobei der Schlauch (17) oder das Röhrchen vorzugsweise als ein Blutungsdrain, ein Bülaudrain, eine PEG-Sonde, eine suprapubische Ableitung oder eine Wunddrainage nach einem chirurgischen Eingriff, oder als eine andere Drainage ausgebildet ist.

14. Anordnung nach Anspruch 13, wobei der Schlauch (17) und/oder das Röhrchen an dessen äusseren Oberfläche wenigstens in einem Befestigungsbereich (19) aufgeraut und/oder mit Kerben (20), Vertiefungen oder Wölbungen ausgebildet ist, wobei der Schlauch(17) oder das Röhrchen mit dem Befestigungsmittel am Befestigungsbereich (19) gehalten ist.
